(19) Europäisches Patentamt
European Patent Office
Office européen des brevets

(11) Veröffentlichungsnummer: **0 097 862**
**B1**

# (12) EUROPÄISCHE PATENTSCHRIFT

(45) Veröffentlichungstag der Patentschrift: **06.08.86**

(51) Int. Cl.⁴: **C 07 D 319/20**, A 01 N 47/36

(21) Anmeldenummer: **83105764.1**

(22) Anmeldetag: **13.06.83**

(54) **Substituierte Benzyl-(thio)harnstoffe, Verfahren zu ihrer Herstellung und ihre Verwendung als Schädlingsbekämpfungsmittel.**

(30) Priorität: **24.06.82 DE 3223505**

(43) Veröffentlichungstag der Anmeldung: **11.01.84 Patentblatt 84/2**

(45) Bekanntmachung des Hinweises auf die Patenterteilung: **06.08.86 Patentblatt 86/32**

(84) Benannte Vertragsstaaten: **AT BE CH DE FR GB IT LI NL**

(56) Entgegenhaltungen: **EP - A - 0 042 533**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Marhold, Albrecht, Dr., Carl-Duisberg-Strasse 329, D-5090 Leverkusen 1 (DE)**
Erfinder: **Sirrenberg, Wilhelm, Dr., Wuppertaler Strasse 21, D-4322 Sprockhoevel (DE)**
Erfinder: **Klauke, Erich, Dr., Eichendorffweg 8, D-5068 Odenthal (DE)**
Erfinder: **Hammann, Ingeborg, Dr., Lutherstrasse 22, D-4330 Mülheim/Ruhr (DE)**
Erfinder: **Becker, Benedikt, Dr., Dürerring 8, D-4030 Ratingen 1 (DE)**
Erfinder: **Krehan, Ingomar, Dr., Ludwig-Jahn-Strasse 54, D-5000 Köln 40 (DE)**
Erfinder: **Stendel, Wilhelm, Dr., In den Birken 55, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft neue N-Fluoralkylendioxy-phenyl -N'- benzoyl-(thio)harnstoffe, Verfahren zu deren Herstellung und deren Verwendung als Schädlingsbekämpfungsmittel, insbesondere als Insektizide.

Es ist bereits bekannt geworden, dass bestimmte Benzoylharnstoffe, wie z.B. N-(4-Chlorphenyl) -N'- (2,6-difluorbenzoyl)-harnstoff, N-(4-Trifluor-methoxy-phenyl) -N'- (2-chlor-benzol)-harnstoff und N-(2,2,4,4-Tetrafluor -1,3-benzodioxin-6-yl) -N'- (2-chlor-benzoyl)-harnstoff, insektizide Eigenschaften aufweisen (vgl. Deutsche Offenlegungsschriften 2 123 236, 2 601 780, 2 637 947 und 3 023 328).

Es wurden nun neue substituierte N-Fluoralkylendioxyphenyl -N'- benzoyl-(thio)harnstoffe der Formel I

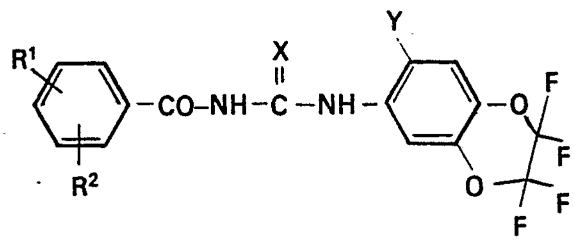

gefunden, in welcher

$R^1$ für Wasserstoff, Halogen oder Alkyl mit 1 bis 6 Kohlenstoffatomen steht;

$R^2$ für Halogen, Alkyl mit 1 bis 6 Kohlenstoffatomen steht;

X für Sauerstoff oder Schwefel steht; und

Y für Wasserstoff, Halogen, Alkyl mit 1 bis 6 Kohlenstoffatomen oder Halogenalkyl mit 1 bis 6 Kohlenstoffatomen steht, wobei die Verbindung ausgenommen sein soll, in welcher $R^1$ für Wasserstoff, $R^2$ für 2-Fluor, X für Schwefel und Y für Wasserstoff stehen.

Man erhält erfindungsgemäss die neuen Verbindungen der Formel (I), wenn man

a) substituierte Benzoyl-iso(thio)cyanate der Formel II

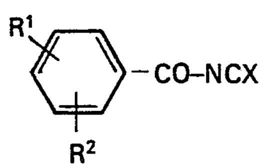

(II)

in welcher

$R^1$, $R^2$ und X die oben angegebenen Bedeutungen haben, mit Fluoralkylendioxy-anilinen der Formel III

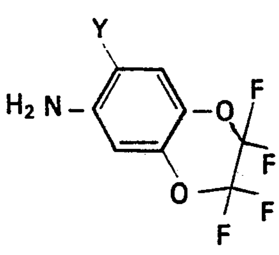

(III)

in welcher

Y die oben angegebene Bedeutung hat, gegebenenfalls in Gegenwart eines Verdünnungsmittels umsetzt oder

b) substituierte Benzamide der Formel IV

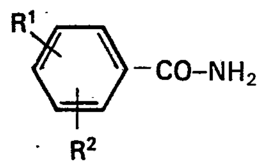

(IV)

in welcher

$R^1$ und $R^2$ die oben angegebenen Bedeutungen haben, mit Fluoralkylendioxy-phenyl-iso(thio)cyanaten der Formel V

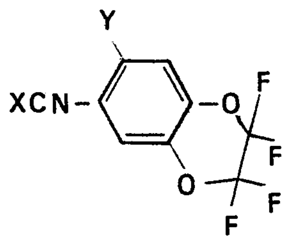

(V)

in welcher

X und Y die oben angegebene Bedeutungen haben, gegebenenfalls unter Verwendung eines Verdünnungsmittels umsetzt, und die gebildeten Endprodukte der Formel I isoliert.

Die neuen Verbindungen der Formel (I) verfügen über Eigenschaften, die ihre Verwendung als Schädlingsbekämpfungsmittel ermöglichen, insbesondere zeichnen sie sich durch eine hervorragende insektizide Wirksamkeit aus.

Überraschenderweise zeigen die erfindungsgemässen N-Fluoralkylendioxy-phenyl -N'- benzoyl-harnstoffe der Formel (I) vorteilhaftere Eigenschaften als die aus dem Stand der Technik bekannten Verbindungen.

In der Definition von $R^1$, $R^2$ und Y bedeutet Alkyl geradkettiges oder verzweigtes Alkyl mit vorzugsweise 1 bis 4 Kohlenstoffatomen.

Genannt seien Methyl, Ethyl, n- und i-Propyl, n-, i- und t-Butyl, n-Pentyl und n-Hexyl. Bevorzugt seien Methyl und Ethyl, insbesondere Methyl genannt.

In Halogenalkyl Y hat die Alkylgruppe die gleiche Bedeutung wie bei Alkyl $R^1$ und Y, wobei die Alkylgruppe druch 1 oder mehrere, vorzugsweise 1 bis 5, insbesondere 1 bis 3 gleiche oder verschiedene Halogenatome substituiert sein kann. Ganz besonders bevorzugt ist die $CF_3$-Gruppe.

Alkylthio $R^2$ steht für Alkylthio mit insbesondere 1 bis 4 Kohlenstoffatomen, wobei Methylthio genannt sei.

X steht vorzugsweise für Sauerstoff.

Y steht vorzugsweise für Wasserstoff.

$R^1$ und $R^2$ können beliebige Positionen des Phenylringes einnehmen. Bevorzugt stehen sie in 2,6-Stellung, 2,5-Stellung, 2,4-Stellung oder 3,4-Stellung, insbesondere in 2,6- oder 2,4- oder

2,5-Stellung, ganz besonders bevorzugt in 2,6-Stellung. Falls $R^1$ für Wasserstoff steht, wird für $R^2$ die 2-, 3- und 4-Positionen bevorzugt, wobei die 2-Stellung besonders bevorzugt wird.

Halogen $R^1$, $R^2$ und Y steht für Fluor, Chlor, Brom oder Jod, vorzugsweise für Fluor, Chlor und Brom, insbesondere für Fluor und Chlor.

Bevorzugte Verbindungen der Formel I sind solche, in welchen die Definition der Reste folgende Bedeutungen haben:

$R^1$ steht für Wasserstoff, Halogen oder Alkyl mit 1 bis 6 Kohlenstoffatomen;

$R^2$ steht für Halogen oder Alkyl mit 1 bis 6 Kohlenstoffatomen;

X steht für Sauerstoff oder Schwefel;

Y steht für Wasserstoff, Halogen, Alkyl mit 1 bis 6 Kohlenstoffatomen oder Halogenalkyl mit 1 bis 6 Kohlenstoffatomen und 1 bis 5 Halogenatomen, wobei die Verbindung ausgenommen werden soll, in welcher $R^1$ für Wasserstoff, $R^2$ für 2-Fluor, X für Schwefel und Y für Wasserstoff stehen.

Die Stellung von $R^1$ und $R^2$ entspricht hierbei den vorherigen Erläuterungen.

In einer besonderen Ausführungsform der Erfindung hat die Definition der Reste in den Verbindungen der Formel (I) die folgende Bedeutung:

$R^1$ steht für Wasserstoff, Fluor, Chlor, Brom, Jod oder Methyl, vorzugsweise für Wasserstoff, Fluor oder Chlor;

$R^2$ steht für Fluor, Chlor, Brom, Jod oder Methyl, vorzugsweise für Fluor oder Chlor;

X steht für Sauerstoff oder Schwefel, vorzugsweise für Sauerstoff; und

Y steht für Wasserstoff, Chlor, Methyl oder Trifluormethyl, vorzugsweise für Wasserstoff, wobei die Verbindung ausgenommen sein soll, in welcher $R^1$ für Wasserstoff, $R^2$ für 2-Fluor, X für Schwefel und Y für Wasserstoff stehen.

Die Stellung von $R^1$ und $R^2$ entspricht hierbei den obigen Erläuterungen.

Hierbei sind besonders bevorzugt Verbindungen der Formel I, in welcher

$R^1$ für Wasserstoff, Fluor oder Chlor steht;

$R^2$ für Fluor oder Chlor steht;

X für Sauerstoff steht; und

Y für Wasserstoff steht.

Die Stellung von $R^1$ und $R^2$ entspricht hierbei den obigen Erläuterungen.

Verwendet man beispielsweise als Ausgangsstoffe bei Verfahren (a) 2-Chlor-benzoyl-isocyanat und 3,4-(Tetrafluorethylendioxy)-anilin sowie bei Verfahren (b) 2,6-Fluor-benzamid und 3,4- (Tetrafluorethylendioxy)- phenylisocyanat, so können die entsprechenden Reaktionen durch folgende Formelschemata skizziert werden.:

a)

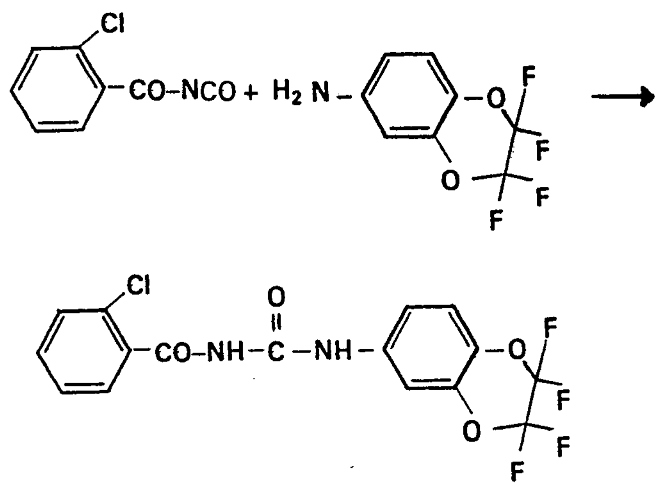

b)

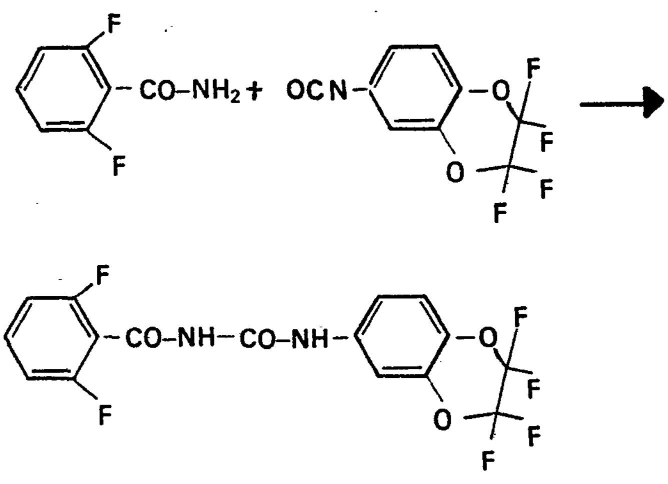

Die bei den Herstellungsverfahren (a) und (b) zu verwendenden Ausgangsstoffe sind durch die Formeln (II) und (III) bzw. (IV) und (V) definiert.

Die als Ausgangsverbindungen zu verwendenden Benzoesäureamide (IV) und die entsprechenden Benzoyl-iso(thio)-cyanate (II) sind bekannt oder können analog bekannten Verfahren nach allgemein üblichen Methoden hergestellt werden (vgl. z.B. J. Org. Chem. 30, (1965), 4306–4307 und DE-AS 1 215 144).

Als Beispiele seien genannt:

2-Fluor-, 2-Chlor-, 2-Brom-, 2-Jod-, 2-Methyl-, 2,6-Difluor-, 2,6-Dichlor-, 2,6-Dibrom-, 2-Chlor-6-fluor-, 2-Chlor-4-fluor- und 2-Chlor-5-fluorbenzoesäureamid, 2-Fluor-, 2-Chlor-, 2-Brom-, 2-Jod-, 2-Methyl-, 2,6-Difluor-, 2,6-Dichlor-, 2,6-Dibrom- und 2-Chlor-6-fluor-, 2-Chlor-4-fluor- und 2-Chlor-5-fluorbenzoyl-isocyanat sowie 2-Fluor-, 2-Chlor-, 2-Brom-, 2-Jod-, 2-Methyl-, 2,6-Difluor-, 2,6-Dichlor-, 2,6-Dibrom- und 2-Chlor-6-fluor -2- Chlor-4-fluor- und 2-Chlor-5-fluorbenzoylisothiocyanat.

Die weiterhin als Ausgangsstoffe zu verwendenden Fluoralkylendioxy-aniline (III) und die entsprechenden Fluoralkylendioxy- phenyl- iso (thio)cyanate (V) sind ebenfalls bekannt oder können analog bekannten Verfahren nach allgemein üblichen Methoden hergestellt werden (vgl. Deutsche Offenlegungsschrift 2 848 531).

Als Beispiele seien genannt:

6-Amino-, 6-Isocyanato- und 6-Isothiocyanato -2,2,3,3- tetrafluor -1,4- benzodioxin,

6-Amino-, 6-Isocyanato- und 6-Isothiocyanato -7- chlor -2,2,3,3- tetrafluor -1,4- benzodioxin,

6-Amino-, 6-Isocyanato- und 6-Isothiocyanato -7- trifluormethyl -2,2,3,3- tetrafluor -1,4-benzodioxin.

Die Verfahrensvarianten zur Herstellung der neuen N-Fluoralkylendioxy-phenyl -N'- benzoyl-(thio)harnstoffe werden vorzugsweise unter Verwendung von Verdünnungsmitteln durchgeführt.

Als Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glycoldimethylether und Diglycoldimethylether, Tetrahydrofuran und Dioxan, Ketone, wie Aceton, Methyl-ethyl-, Methyl-isopropyl- und Methylisobutylketon, Ester, wie Essigsäuremethylester und -ethylester, Nitrile, wie z.B. Acetonitril und Propionitril, Amide, wie z.B. Dimethylformamid, Dimethylacetamid und N-Methyl-pyrrolidon, sowie Dimethylsulfoxid, Tetramethylensulfon und Hexamethylphosphorsäuretriamid.

Die Reaktionstemperatur kann innerhalb eines grösseren Bereichs variiert werden. Im allgemeinen arbeitet man zwischen 20 und 180 °C, vorzugsweise bei 60 bis 120 °C. Die erfindungsgemässen Verfahrensvarianten werden im allgemeinen bei Normaldruck durchgeführt.

Zur Durchführung der erfindungsgemässen Verfahrensvarianten werden die Ausgangsstoffe gewöhnlich in äquimolaren Mengen eingesetzt. Ein Überschuss der einen oder anderen Reaktionskomponente bringt keine wesentlichen Vorteile. Die Umsetzung wird im allgemeinen in einem geeigneten Verdünnungsmittel durchgeführt und das Reaktionsgemisch wird mehrere Stunden bei der erforderlichen Temperatur gerührt. Danach lässt man das Reaktionsgemisch abkühlen und saugt im Falle, dass die Endprodukte im verwendeten Lösungsmittel schwer löslich sind, vom auskristallisierten Produkt ab. Ansonsten erfolgt die Isolierung und gegebenenfalls Reinigung nach allgemein üblichen Methoden, z.B. durch Abdampfen des Lösungsmittels (gegebenenfalls unter vermindertem Druck). Zur Charakterisierung dient der Schmelzpunkt, das NMR-Spektrum und die Elementaranalyse.

Wie bereits dargelegt, betrifft die vorliegende Erfindung ausser den neuen Verbindungen der Formel I und deren Herstellung auch Schädlingsbekämpfungsmittel, welche Verbindungen der Formel I enthalten, die Herstellung dieser Schädlingsbekämpfungsmittel und ihre Verwendung. Die erfindungsgemässen Verbindungen der Formel I zeigen auch eine fungizide Wirksamkeit, was ihren Wert bei der Verwendung als Schädlingsbekämpfungsmittel im Pflanzenschutz steigert.

Die Wirkstoffe eignen sich bei guter Pflanzenverträglichkeit zur Bekämpfung von tierischen Schädlingen, insbesondere Insekten, Spinnentieren und Nematoden, ganz besonders bevorzugt zur Bekämpfung von Insekten, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bamisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: stark polare Lösungsmittel, wie Dimethylformamid und Dimethylsulfoxid; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-ether, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Spurennährstoffe wie Salze von Eisen, Mangan, Bor,

Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemässen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepassten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemässen Wirkstoffe eignen sich auch zur Bekämpfung von Ekto- und Endoparasiten vorzugsweise von ektoparasitierenden Insekten auf dem Gebiet der Tierhaltung und der Tierzucht.

Die Anwendung der erfindungsgemässen Wirkstoffe geschieht hier in bekannter Weise, wie durch orale Anwendung, durch dermale Anwendung in Form beispielsweise des Tauchens (Dippen), Sprühens (Sprayen), Aufgiessens (pour-on and spot-on) und des Einpuderns.

Die erfindungsgemässen neuen Verbindungen der Formel I können demgemäss auch besonders vorteilhaft in der Viehhaltung (z.B. Rinder, Schafe, Schweine und Geflügel wie Hühner, Gänse usw.) eingesetzt werden. In einer bevorzugten Ausführungsform der Erfindung werden den Tieren die neuen Verbindungen, gegebenenfalls in geeigneten Formulierungen (vgl. oben) und gegebenenfalls mit dem Trinkwasser oder Futter oral verabreicht. Da eine Ausscheidung im Kot in wirksamer Weise erfolgt, lässt sich auf diese Weise sehr einfach die Entwicklung von Insekten im Kot der Tiere verhindern. Die jeweils geeigneten Dosierungen und Formulierungen sind insbesondere von der Art und dem Entwicklungsstadium der Nutztiere und auch vom Befallsdruck der Insekten abhängig und lassen sich nach den üblichen Methoden leicht ermitteln und festlegen. Die neuen Verbindungen können bei Rindern z.B. in Dosiermengen von 0,01 bis 1 mg/kg Körpergewicht eingesetzt werden.

Die insektizide Wirksamkeit der neuen erfindungsgemässen Verbindungen ergibt sich aus den folgenden Beispielen:

Beispiel A

Phaedon-Larven Test

Lösungsmittel: 15 Gewichtsteile Dimethylformamid

Emulgator: 1 Gewichtsteil Alkylarylpolyglykolether

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100%, dass alle Larven abgetötet wurden; 0% bedeutet, dass keine Larven abgetötet wurden.

Bei diesem Test zeigten z.B. bei einem Versuch mit einer Wirkstoffkonzentration von 0,01% die Verbindungen Nr. 1, 2, 3, 4, 5, 6 und 14 eine Abtötung von 100% nach 10 Tagen.

Beispiel B

Laphygma-Test

Lösungsmittel: 15 Gewichtsteile Dimethylformamid

Emulgator: 1 Gewichtsteil Alkylarylpolyglykolether

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Laphygma frugiperda) besetzt, solange die Blätter noch feucht sind.

Nach der gewünschten Zeit wird die Abtötung in % bestimmt. Dabei bedeutet 100%, dass alle Raupen abgetötet wurden; 0% bedeutet, dass keine Raupen abgetötet wurden.

Bei diesem Test zeigten z.B. bei einem Versuch mit einer Wirkstoffkonzentration von 0,001% die Verbindungen Nr. 1, 3, 4 und 14 eine Abtötung von 100% nach 7 Tagen.

Beispiel C

Heliothis armigera-Test

Lösungsmittel: 15 Gewichtsteile Dimethylformamid

Emulgator: 1 Gewichtsteil Alkylarylpolyglykolether

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) werden durch Tauchen in die Wirkstoffzubereitung der gewünschten Konzentration behandelt und mit der Tabakknospenraupe (Heliothis armigera) besetzt, solange die Blätter noch feucht sind.

Nach der gewünschten Zeit wird die Abtötung in % bestimmt. Dabei bedeutet 100%, dass alle Raupen abgetötet wurden; 0% bedeutet, dass keine Raupen abgetötet wurden.

Bei diesem Test zeigte z.B. bei einem Versuch mit einer Wirkstoffkonzentration von 0,00016 die Verbindung aus Beispiel 5 eine Abtötung von 100% nach 7 Tagen.

Beispiel D

Mückenlarven-Test

Testtiere: Aedes aegypti

Lösungsmittel: Aceton, 99 Gewichtsteile

Emulgator: Benzylhydroxydiphenylglykolether, 1 Gewichtsteil

Zur Herstellung einer zweckmässigen Wirkstoffzubereitung löst man 2 Gew.-Teile Wirkstoff in 1000 Volumenteilen Lösungsmittel, das Emulgator in der oben angegebenen Menge enthält. Die so erhaltene Lösung wird mit Wasser auf die gewünschten geringeren Konzentrationen verdünnt.

Man füllt die wässrigen Wirkstoffzubereitungen der gewünschten Konzentration in Gläser und setzt anschliessend etwa 25 Mückenlarven in jedes Glas ein.

Nach 21 Tagen wird der Abtötungsgrad in % bestimmt. Dabei bedeutet 100%, dass alle Larven abgetötet worden sind. 0% bedeutet, dass überhaupt keine Larven abgetötet worden sind.

Bei diesem Test zeigten z.B. bei einem Versuch mit Verdünnungen von $10^{-3}$ bis $10^{-4}$ ppm die Verbindungen Nr. 1, 3, 4 und 5 eine Abtötung von 100% in 21 Tagen.

Beispiel E

Test mit Lucilia cuprina res.-Larven

Lösungsmittel: 35 Gewichtsteile Ethylenglykolmonomethylether

35 Gewichtsteile Nonylphenolpolyglykolether

Zur Herstellung einer zweckmässigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^2$ Pferdefleisch und 0,5 ml der Wirkstoffzubereitung enthält. Nach 24 Stunden wird der Abtötungsgrad bestimmt.

Bei diesem Test zeigten z.B. bei einem Versuch mit einer Wirkstoffkonzentration von 100 ppm die Verbindungen Nr. 1, 2, 3, 4, 5 und 6 Abtötungsgrade zwischen 50 und 100%.

Die Herstellung der erfindungsgemässen Verbindungen der Formel I soll durch die folgenden Herstellungsbeispiele erläutert werden:

Beispiel 1

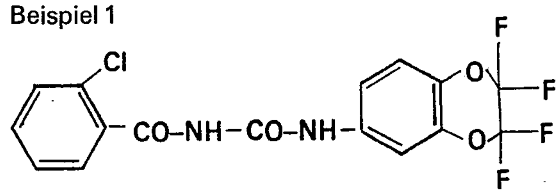

4,6 g 7 - Amino - 2,2,3,3 - tetrafluorbenzo-1,4-dioxen werden in 60 ml trockenem Toluol gelöst und bei 60 °C unter Feuchtigkeitsausschluss mit 3,64 g 2-Chlorbenzoylisocyanat versetzt. Der Ansatz rührt eine Stunde bei 80 °C und wird dann auf Raumtemperatur abgekühlt. Das ausgefallene Produkt wird abgesaugt und im Vakuum getrocknet. Man erhält 8 g 1- (2-Chlorbenzoyl) -3-(2,2,3,3-tetrafluorbenzo -1,4- dioxen)- harnstoff vom Schmelzpunkt 198 °C.

Analog werden Verbindungen der folgenden Tabellen erhalten:

Tabelle I:

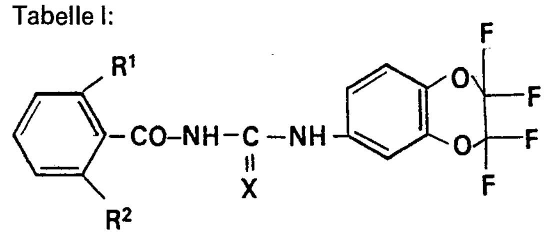

| Beisp. Nr. | $R^{1'}$ | $R^{2'}$ | X | Schmp. (°C) |
|---|---|---|---|---|
| 2 | Cl | Cl | O | 202 |
| 3 | Cl | F | O | 197 |
| 4 | H | Br | O | 199 |
| 5 | F | F | O | 207 |
| 6 | H | F | O | 169 |
| 7 | H | $C_2H_5$ | O | 137 |
| 8 | H | $SCH_3$ | O | 197 |
| 9 | F | F | S | 189 |
| 10 | Cl | F | S | 203 |
| 11 | H | Br | S | 172 |
| 12 | H | $CH_3$ | S | 154 |
| 13 | Cl | Cl | S | 208 |

Tabelle II:

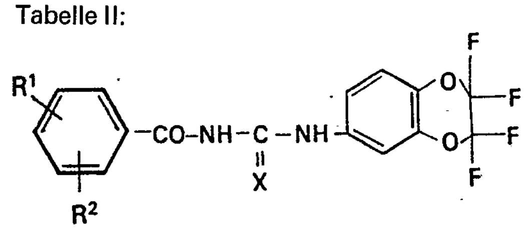

| Beisp. Nr. | $R^{1'}$ | $R^{2'}$ | X | Schmp. (°C) |
|---|---|---|---|---|
| 14 | 2-Cl | 4-F | O | 157 |
| 15 | 2-Cl | 5-F | O | 182 |
| 16 | H | 3-Cl | O | 194 |
| 17 | H | 4-Cl | O | 224 |
| 18 | 3-Cl | 4-Cl | O | 197 |

Die Herstellung der Ausgangsverbindungen der Formel (II) soll anhand des folgenden Beispiels erläutert werden (Y = Wasserstoff).

Stufe 1:

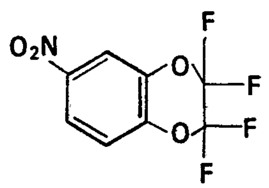

In einem VA-Autoklaven mit Rührer und Rückflusskühler werden 250 ml Fluorwasserstoff, 3 ml Antimonpentachlorid und 330 g 7-Nitro-2,2,3-trifluor -3- chlor-benzo -1,4- dioxen vorgelegt, 3 bar Stickstoff aufgedrückt und anschliessend auf 125 °C erhitzt. Der entstehende Chlorwasserstoff wird über ein Regelventil am Rückfluss bei 20 bar entspannt. Nach 10 Stunden Reaktionszeit wird der überschüssige Fluorwasserstoff abdestilliert und der Rückstand mit Wasserdampf destilliert. Man erhält 265 g Produkt ($n_D^{20}$: 1.4821), das nach GC-Analyse zu 79% aus 7-Nitro-2,2,3,3-tetrafluor- benzo-1,4-dioxen und 21% Ausgangsmaterial besteht. Durch fraktionierte Destillation wird reines 7-Nitro-2,2,3,3-tetrafluor- benzo-1,4-dioxen vom Siedepunkt Kp: 98–100 °C/16 mbar und Brechungsindex $n_D^{20}$: 1,4750 erhalten.

Stufe 2:

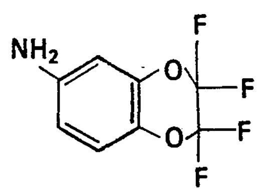

In einer Hydrierapparatur werden 52 g 7-Nitro-2,2,3,3- tetrafluorbenzo -1,4- dioxen in 180 ml Methanol in Gegenwart von 5 g Raney-Nickel bei 25–45 °C mit 30–50 bar Wasserstoff hydriert. Nach dem Entspannen wird der Katalysator abfiltriert und die Lösung destilliert. Man erhält bei Kp: 95–97 °C/16 mbar 40 g 7-Amino -2,2,3,3- tetrafluorbenzo -1,4- dioxen vom Schmelzpunkt 32 °C.

Die übrigen Ausgangsstoffe können wie oben erläutert nach allgemein üblichen Methoden und Verfahren erhalten werden.

**Patentansprüche**

1. Verbindungen der allgemeinen Formel I

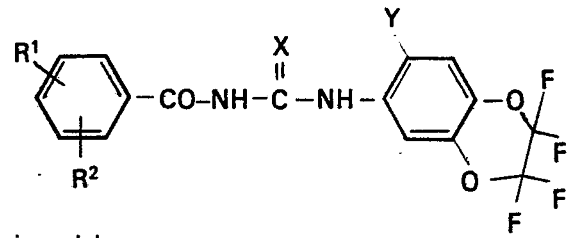

in welcher,

$R^1$ für Wasserstoff, Halogen oder Alkyl mit 1 bis 6 Kohlenstoffatomen steht;

$R^2$ für Halogen, Alkyl mit 1 bis 6 Kohlenstoffatomen oder Alkylthio mit 1 bis 6 Kohlenstoffatomen steht;

X für Sauerstoff oder Schwefel steht; und

Y für Wasserstoff, Halogen, Alkyl mit 1 bis 6 Kohlenstoffatomen oder Halogenalkyl mit 1 bis 6 Kohlenstoffatomen steht, wobei die Verbindung ausgenommen werden soll, in welcher $R^1$ für Wasserstoff, $R^2$ für 2-Fluor, X für Schwefel und Y für Wasserstoff stehen.

2. Verbindungen gemäss Anspruch 1, in welchen

$R^1$ für Wasserstoff, Halogen oder Alkyl mit 1 bis 6 Kohlenstoffatomen steht;

$R^2$ für Halogen oder Alkyl mit 1 bis 6 Kohlenstoffatomen steht;

X für Sauerstoff oder Schwefel steht;

Y für Wasserstoff, Halogen, Alkyl mit 1 bis 6 Kohlenstoffatomen oder Halogenalkyl mit 1 bis 6 Kohlenstoffatomen und 1 bis 5 Halogenatomen steht, wobei die Verbindung ausgenommen werden soll, in welcher $R^1$ für Wasserstoff, $R^2$ für 2-Fluor, X für Schwefel und Y für Wasserstoff stehen.

3. Verbindungen gemäss Anspruch 1, in welchen

$R^1$ für Wasserstoff, Fluor, Chlor, Brom, Jod oder Methyl steht;

$R^2$ für Fluor, Chlor, Brom, Jod oder Methyl steht;

X für Sauerstoff oder Schwefel steht; und

Y für Wasserstoff, Chlor, Methyl oder Trifluormethyl steht, wobei die Verbindung ausgenommen werden soll, in welcher $R^1$ für Wasserstoff, $R^2$ für 2-Fluor, X für Schwefel und Y für Wasserstoff stehen.

4. Verbindungen gemäss Anspruch 1, in welchen

$R^1$ für Wasserstoff, Fluor oder Chlor steht;

$R^2$ für Fluor oder Chlor steht;

X für Sauerstoff steht; und

Y für Wasserstoff steht.

5. Verbindung der Formel

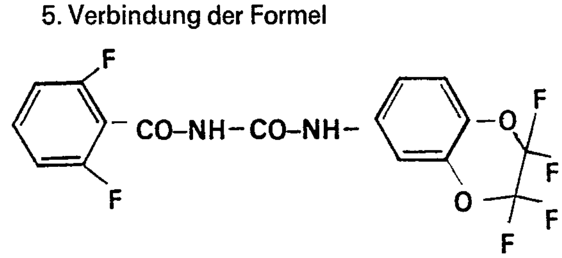

6. Verbindung der Formel

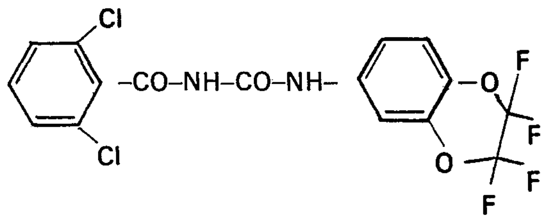

7. Verfahren zur Herstellung der Verbindungen der allgemeinen Formel I

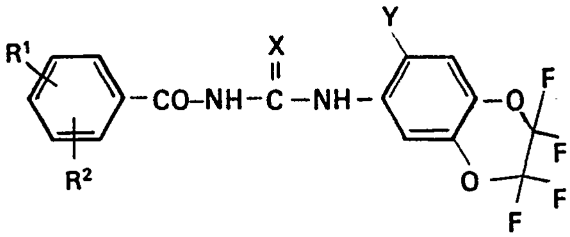

in welcher,

$R^1$ für Wasserstoff, Halogen oder Alkyl mit 1 bis 6 Kohlenstoffatomen steht;

$R^2$ für Halogen, Alkyl mit 1 bis 6 Kohlenstoffatomen oder Alkylthio mit 1 bis 6 Kohlenstoffatomen steht;

X für Sauerstoff oder Schwefel steht; und

Y für Wasserstoff, Halogen, Alkyl mit 1 bis 6 Kohlenstoffatomen oder Halogenalkyl mit 1 bis 6 Kohlenstoffatomen steht, wobei die Verbindung ausgenommen werden soll, in welcher $R^1$ für Wasserstoff, $R^2$ für 2-Fluor, X für Schwefel und Y für Wasserstoff stehen, dadurch gekennzeichnet, dass man

(a) substituierte Benzoyl-iso(thio)cyanate der Formel II

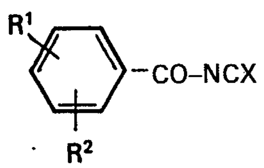

(II)

in welcher

$R^1$, $R^2$ und X die oben angegebenen Bedeutungen haben, mit Fluoralkylendioxy-anilinen der Formel III

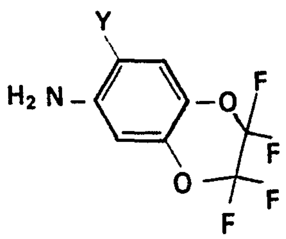

(III)

in welcher

Y die oben angegebene Bedeutung hat, gegebenenfalls in Gegenwart eines Verdünnungsmittels umsetzt oder

(b) substituierte Benzamide der Formel IV

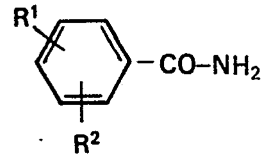

(IV)

in welcher

$R^1$ und $R^2$ die oben angegebenen Bedeutungen haben, mit Fluoralkylendioxy-phenyl-iso(thio) cyanaten der Formel V

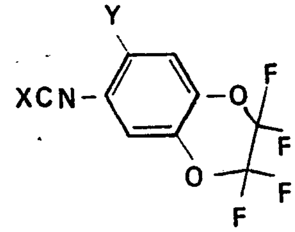

(V)

in welcher

X und Y die oben angegebenen Bedeutungen haben, gegebenenfalls unter Verwendung eines Verdünnungsmittels umsetzt, und die gebildeten Endprodukte der Formel I isoliert.

8. Schädlingsbekämpfungsmittel, gekennzeichnet durch den Gehalt an mindestens einer Verbindung gemäss den Ansprüchen 1 bis 6.

9. Herstellung der Schädlingsbekämpfungsmittel gemäss Anspruch 8, dadurch gekennzeichnet, dass man mindestens eine Verbindung gemäss den Ansprüchen 1 bis 6 mit inerten Streck- und Verdünnungsmitteln und gegebenenfalls mit oberflächenaktiven Stoffen mischt.

10. Verwendung der Verbindungen der Ansprüche 1 bis 6 zur Bekämpfung von Schädlingen, die in der Landwirtschaft, in Forsten, im Vorrats- und Materialschutz sowie auf dem Hygienesektor vorkommen.

11. Verbindungen der allgemeinen Formel I gemäss Anspruch 1 zur Bekämpfung von Ekto- und Endoparasiten.

**Revendications**

1. Composé de formule générale (I)

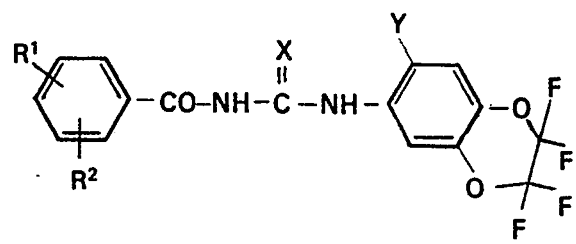

dans laquelle:

$R^1$ représente un atome d'hydrogène ou d'halogène ou un groupe alkyle ayant 1 à 6 atomes de carbone;

$R^2$ représente un atome d'halogène, un groupe alkyle ayant 1 à 6 atomes de carbone ou alkylthio ayant 1 à 6 atomes de carbone;

X représente un atome d'oxygène ou de soufre; et

Y représente un atome d'hydrogène ou d'halogène, un groupe alkyle ayant 1 à 6 atomes de carbone ou halogénoalkyle ayant 1 à 6 atomes de carbone, à l'exclusion du composé dans lequel $R^1$ représente un atome d'hydrogène, $R^2$ représente un atome de fluor en position 2, X représente un atome de soufre et Y un atome d'hydrogène.

2. Composés selon la revendication 1, dans lesquels:

$R^1$ représente un atome d'hydrogène ou d'halogène ou un groupe alkyle ayant 1 à 6 atomes de carbone;

$R^2$ représente un atome d'halogène ou un groupe alkyle ayant 1 à 6 atomes de carbone;

X représente un atome d'oxygène ou de soufre;

Y représente un atome d'hydrogène ou d'halogène, un groupe alkyle ayant 1 à 6 atomes de carbone ou halogénoalkyle ayant 1 à 6 atomes de carbone et 1 à 5 atomes d'halogène, à l'exclusion du composé dans lequel $R^1$ représente un atome d'hydrogène, $R^2$ représente un atome de fluor en position 2, X représente un atome de soufre et Y un atome d'hydrogène.

3. Composés selon la revendication 1, dans lesquels:

$R^1$ représente un atome d'hydrogène, de fluor, de chlore, de brome ou d'iode ou un groupe méthyle;

$R^2$ représente un atome de fluor, de chlore, de brome ou d'iode ou un groupe méthyle;

X représente un atome d'oxygène ou de soufre; et

Y représente un atome d'hydrogène ou de chlore ou un groupe méthyle ou trifluorméthyle, à l'exclusion du composé dans lequel $R^1$ représente un atome d'hydrogène, $R^2$ représente un atome de fluor en position 2, X représente un atome de soufre et Y un atome d'hydrogène.

4. Composés selon la revendication 1, dans lesquels:

$R^1$ représente un atome d'hydrogène, de fluor ou de chlore;

$R^2$ représente un atome de fluor ou de chlore;

X représente un atome d'oxygène; et

Y représente un atome d'hydrogène.

5. Composé de formule:

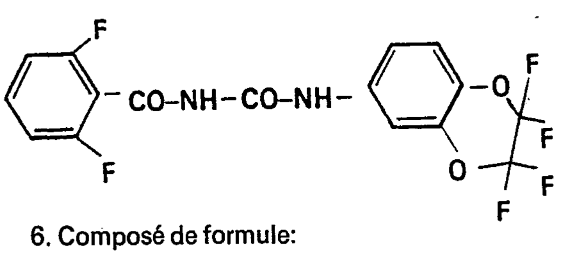

6. Composé de formule:

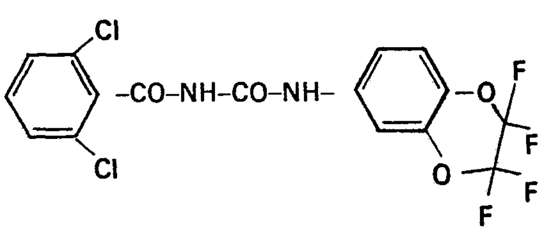

7. Procédé pour préparer les composés de formule générale (I):

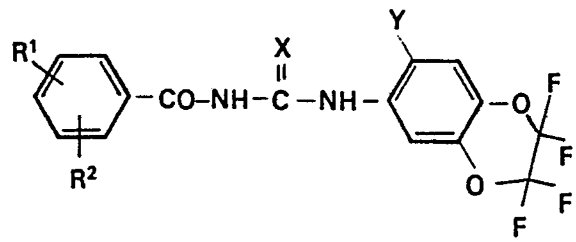

dans laquelle:

$R^1$ représente un atome d'hydrogène ou d'halogène ou un groupe alkyle ayant 1 à 6 atomes de carbone;

$R^2$ représente un atome d'halogène, un groupe alkyle ayant 1 à 6 atomes de carbone ou alkylthio ayant 1 à 6 atomes de carbone;

X représente un atome d'oxygène ou de soufre; et

Y représente un atome d'hydrogène ou d'halogène, un groupe alkyle ayant 1 à 6 atomes de carbone ou halogénoalkyle ayant 1 à 6 atomes de carbone, à l'exclusion du composé dans lequel $R^1$ représente un atome d'hydrogène, $R^2$ représente un atome de fluor en position 2, X représente un atome de soufre et Y un atome d'hydrogène; caractérisé en ce que:

(a) on fait réagir, éventuellement en présence d'un diluant, des iso(thio)cyanates de benzoyles substitués, de formule (II):

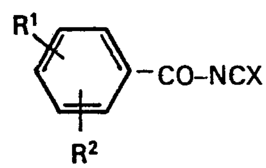

(II)

dans laquelle:

$R^1$, $R^2$ et X ont les sens indiqués ci-dessus; avec des fluoralkylènedioxy-anilines de formule (III)

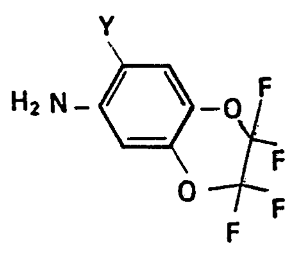

(III)

dans laquelle:

Y a le sens indiqué ci-dessus, ou

(b) on fait réagir, éventuellement en utilisant un diluant, des benzamides substitués de formule (IV):

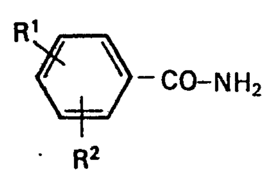

(IV)

dans laquelle:

$R^1$ et $R^2$ ont les sens indiqués ci-dessus, avec des iso(thio)cyanates de fluoralkylènedioxyphényles de formule (V):

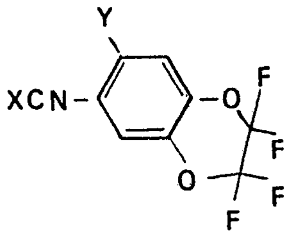

(V)

dans laquelle:

X et Y ont les sens indiqués ci-dessus, et l'on isole les produits finals formés, de formule (I).

8. Pesticides, caractérisés en ce qu'ils contiennent au moins un composé selon les revendications 1 à 6.

9. Préparation des pesticides selon la revendication 8, caractérisée en ce qu'on mélange au moins un composé selon les revendications 1 à 6 avec des agents inertes d'allongement et de dilution et éventuellement avec des substances tensioactives.

10. Utilisation des composés des revendications 1 à 6 pour lutter contre les parasites qui apparaissent en agriculture, dans les forêts, pour la protection des réserves et des matières ainsi que dans le secteur de l'hygiène.

11. Composé de formule générale (I) selon la revendication 1 pour combattre des ecto-parasites et des endo-parasites.

**Claims**

1. Compounds of the general formula I

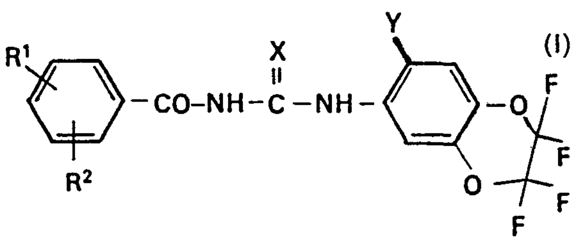

(I)

in which

$R^1$ represents hydrogen, halogen or alkyl with 1 to 6 carbon atoms;

$R^2$ represents halogen, alkyl with 1 to 6 carbon atoms or alkylthio with 1 to 6 carbon atoms;

X represents oxygen or sulphur; and

Y represents hydrogen, halogen, alkyl with 1 to 6 carbon atoms or halogenoalkyl with 1 to 6 carbon atoms, with the exception of the compound in which $R^1$ represents hydrogen, $R^2$ represents 2-fluoro, X represents sulphur and Y represents hydrogen.

2. Compounds according to claim 1, in which

$R^1$ represents hydrogen, halogen or alkyl with 1 to 6 carbon atoms;

$R^2$ represents halogen or alkyl with 1 to 6 carbon atoms;

X represents oxygen or sulphur; and

Y represents hydrogen, halogen, alkyl with 1 to 6 carbon atoms or halogenoalkyl with 1 to 6 carbon atoms and 1 to 5 halogen atoms, with the exception of the compound in which $R^1$ represents hydrogen, $R^2$ represents 2-fluoro, X represents sulfphur and Y represents hydrogen.

3. Compounds according to claim 1, in which

$R^1$ represents hydrogen, fluorine, chlorine, bromine, iodine or methyl;

$R^2$ represents fluorine, chlorine, bromine, iodine or methyl;

X represents oxygen or sulphur; and

Y represents hydrogen, chlorine, methyl or trifluoromethyl, with the exception of the compound in which $R^1$ represents hydrogen, $R^2$ represents 2-fluoro, X represents sulphur and Y represents hydrogen.

4. Compounds according to claim 1, in which

$R^1$ represents hydrogen, fluorine or chlorine;

$R^2$ represents fluorine or chlorine;

X represents oxygen; and

Y represents hydrogen.

5. Compound of the formula

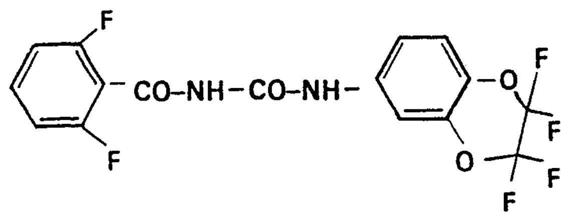

6. Compound of the formula

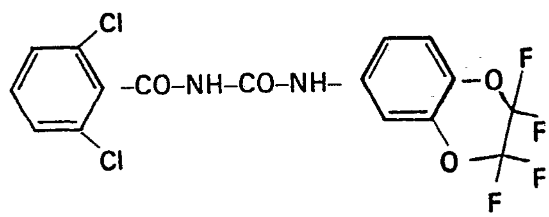

7. Process for the preparation of the compounds of the general formula I

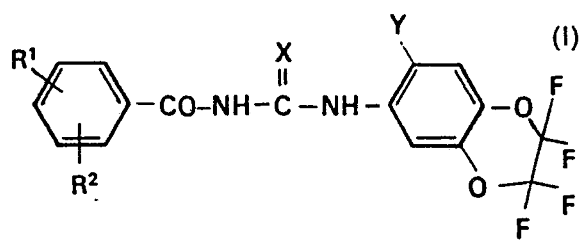

(I)

in which

$R^1$ represents hydrogen, halogen or alkyl with 1 to 6 carbon atoms;

$R^2$ represents halogen, alkyl with 1 to 6 carbon atoms or alkylthio with 1 to 6 carbon atoms;

X represents oxygen or sulphur; and

Y represents hydrogen, halogen, alkyl with 1 to 6 carbon atoms or halogenoalkyl with 1 to 6 carbon atoms, with the exception of the compound in which $R^1$ represents hydrogen, $R^2$ represents 2-fluoro, X represents sulphur and Y represents hydrogen, characterised in that

(a) substituted benzoyl-iso(thio)cyanates of the formula II

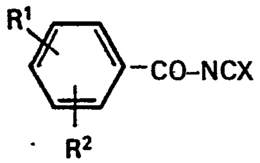

(II)

in which
$R^1$, $R^2$ and X have the abovementioned meanings, are reacted with fluoroalkylenedioxy-anilines of the formula III

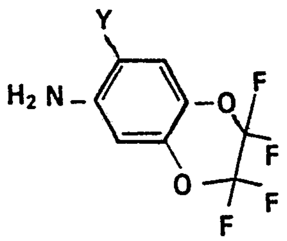

(III)

in which
Y has the abovementioned meaning, if appropriate in the presence of a diluent or
(b) substituted benzamides of the formula IV

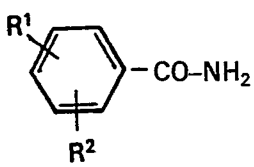

(IV)

in which
$R^1$ and $R^2$ have the abovementioned meanings, are reacted with fluoroalkylenedioxy-phenyl iso-(thio)cyanates of the formula V

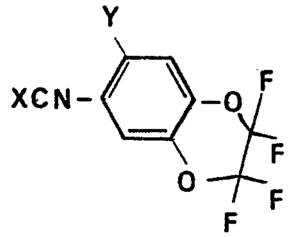

(V)

in which
X and Y have the abovementioned meanings, if appropriate using a diluent, and the end products of the formula I formed are isolated.

8. Agents for combating pests, characterised in that they contain at least one compound according to claims 1 to 6.

9. Preparation of the agents for combating pests according to claim 8, characterised in that at least one compound according to claims 1 to 6 is mixed with inert extenders and diluents and if appropriate with surface-active substances.

10. Use of the compounds of claims 1 to 6 for combating pests which are encountered in agriculture, in woodlands, in the protection of stored products and of materials and in the hygiene sector.

11. Compounds of the general formula I according to claim 1 for combating ecto- and endoparasites.